# EUROPEAN PATENT SPECIFICATION

(11) **EP 4 158 343 B1**
(45) Date of publication and mention of the grant of the patent: **09.04.2025**
(21) Application number: 21731708.0
(22) Date of filing: 31.05.2021
(51) Int. Cl.: G01N 33/543

(54) **MACHINE READABLE DIAGNOSTIC TESTS AND METHODS AND APPARATUS TO MAKE AND/OR PROCESS THE SAME**
MASCHINENLESBARE DIAGNOSTISCHE TESTS UND VERFAHREN UND VORRICHTUNG ZUR HERSTELLUNG UND/ODER VERARBEITUNG DAVON
TESTS DIAGNOSTIQUES LISIBLES PAR MACHINE ET PROCÉDÉS ET APPAREIL POUR EFFECTUER ET/OU TRAITER CEUX-CI

(30) Priority: 29.05.2020 US 202063032102 P
(43) Date of publication of application: 05.04.2023
(60) Divisional application: 25160251.2
(73) Proprietor: Abbott Rapid Diagnostics International Unlimited Company, Dublin 2 (IE)
(72) Inventor: HELLMICH-DUONG, Thanh Tu, 07745 Jena (DE); SUCH, Olaf, 52074 Aachen (DE); BUENNING, Carsten, 68649 Gross-Rohrheim (DE)
(74) Representative: Mathys & Squire
(86) International application number: PCT/EP2021/064583
(87) International publication number: WO 2021/240021

(56) References cited:
- EP-A1- 2 554 992
- KR-A- 20190 005 054
- US-A1- 2012 171 698
- US-A1- 2012 184 462
- US-A1- 2018 319 657

## Description

### FIELD OF THE DISCLOSURE

This disclosure relates generally to biosensors, and, more particularly, to a machine readable diagnostic tests and methods and apparatus to make and/or process the same.

### BACKGROUND

A biosensor (e.g., a lateral flow device, such as a lateral flow assay (LFA)) is a device that is capable of detecting a condition, disease, etc., in a human or animal based on a sample (e.g., a blood sample, a saliva sample, a urine sample, etc.) from the human or animal. LFAs have been used to detect the presence of a target analyte to determine pregnancy, presence of HIV, presence of Ebola, presence of different toxins, etc.
US 2018/319657 A1 describes a multiplex lateral flow device array that can include a number of immobilisation zones arranged in a pattern such that digits can be formed for observation by a user.
EP 2 554 992 A1 describes a multi-assay immunochromatographic chip that can include a number of detection zones in a fixed pattern.
US 2012/184462 A1 describes a lateral flow device with two-dimensional features that can include a number of reagent dots for detecting multiple analytes.
US 2012/171698 A1 describes a microfluidic device that can include a number of capture regions in a fixed pattern.
KR 2019 0005054 A describes a lateral flow test or immunochromatographic device that can include a number of trapping areas.

### BRIEF DESCRIPTION OF THE DRAWINGS

FIG. 1A is an example environment including an example enhanced lateral flow immunoassay generator to generate an enhanced lateral flow immunoassay described in conjunction with examples disclosed herein.
FIG. 1B illustrates an example test grid of the enhanced lateral flow immunoassay of FIG. 1A.
FIG. 2 is block diagram of an implementation of the test grid generator of FIG. 1A.
FIG. 3 is block diagram of an implementation of the enhanced lateral flow immunoassay reader application of FIG. 1A.
FIG. 4 illustrates a flowchart representative of machine readable instructions which may be executed to implement the test grid generator of FIGS. 1A and/or 2.
FIG. 5A-5B illustrates a flowchart representative of machine readable instructions which may be executed to implement enhanced lateral flow immunoassay reader application of FIGS. 1A and/or 3.
FIG. 6 illustrates an example implementation of the enhanced lateral flow immunoassay of FIG. 1A
FIG. 7 is a block diagram of an example processing platform structured to execute the instructions of FIG. 4 to implement the test grid generator of FIGS. 1A and/or 2.
FIG. 8 is a block diagram of an example processing platform structured to execute the instructions of FIG. 5A and/or 5B to implement the enhanced lateral flow immunoassay reader application of FIGS. 1A and/or 3.

The figures are not to scale. Instead, the thickness of the layers or regions may be enlarged in the drawings. In general, the same reference numbers will be used throughout the drawing(s) and accompanying written description to refer to the same or like parts. As used in this patent, stating that any part (e.g., a layer, film, area, region, or plate) is in any way on (e.g., positioned on, located on, disposed on, or formed on, etc.) another part, indicates that the referenced part is either in contact with the other part, or that the referenced part is above the other part with one or more intermediate part(s) located therebetween. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Stating that any part is in "contact" with another part means that there is no intermediate part between the two parts. Although the figures show layers and regions with clean lines and boundaries, some or all of these lines and/or boundaries may be idealized. In reality, the boundaries and/or lines may be unobservable, blended, and/or irregular.

Descriptors "first," "second," "third," etc. are used herein when identifying multiple elements or components which may be referred to separately. Unless otherwise specified or understood based on their context of use, such descriptors are not intended to impute any meaning of priority, physical order or arrangement in a list, or ordering in time but are merely used as labels for referring to multiple elements or components separately for ease of understanding the disclosed examples. In some examples, the descriptor "first" may be used to refer to an element in the detailed description, while the same element may be referred to in a claim with a different descriptor such as "second" or "third." In such instances, it should be understood that such descriptors are used merely for ease of referencing multiple elements or components.

### DETAILED DESCRIPTION

A biosensor or test strip device (e.g., lateral flow immunoassay (LFA)) is a device or diagnostic test strip that includes a first region to obtain a sample (e.g., blood, urine, saliva, etc.) and a second region that changes color when a target analyte corresponding to a particular disease or condition is present in the sample. For example, a user applies the sample to a sample pad of a test strip device, or simply "test strip" (e.g., an LFA, etc.). Once applied, the sample migrates along the test strip to a conjugate pad that contains conjugates (e.g., detectable labels, tags, linkers, antibodies, antigens, etc.) specific to the target analyte. If the sample includes the target analyte, a chemical reaction will occur on the conjugate pad to bind the target analyte with the conjugates. The test strip also includes a test line that contains immobilized antibodies and/or antigens specific to the target analyte, which bind the first set of conjugate molecules (e.g., probe molecules) from the conjugate pad. For example, if the analyte of interest is an antibody, the positive test area includes immobilized antigen. If the analyte of interest is an antigen, the positive test area includes immobilized antibody. The labeled substance or conjugate includes a first binding component that is able to bind the analyte of interest and a second visualization component. Accordingly, when the sample (e.g., including the bounded target analyte) flows to a test zone (e.g., a reaction zone), the antibodies or antigens of the test line bind to the bounded target analyte, thereby immobilizing the target analyte. In some test strips, the immobilized target analytes result in a visual output that identifies that the target analyte was present in the sample. Accordingly, an optical scanner or user can identify whether the target analyte (e.g., corresponding to a condition or disease) is present in the sample based on the color of the test zone.

"Target analyte", "analyte" or "analyte of interest" refers to the compound or the composition to be detected or measured from the sample, which has at least one epitope or binding site. The analyte can be any substance for which there exists a naturally occurring analyte-specific binding member or for which an analyte-specific binding member can be prepared. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), and/or metabolites of or antibodies to any of the above substances. The term "analyte" also includes any antigenic substances, haptens, antibodies, macromolecules, and/or combinations thereof.

"Label" refers to any substance which is capable of producing a signal that is detectable by visual and/or instrumental means. Various labels suitable for use in examples disclosed herein include labels that produce signals through chemical and/or physical means. Examples include enzymes and substrates, chromagens, fluorescent compounds, chemiluminescent compounds, colored or colorable organic polymer latex particles, liposomes, and/or other vesicles containing directly visible substances. In some examples radioactive labels, colloidal metallic particles, and/or colloidal non-metallic particles are employed. In some examples, labels include colloidal gold and latex particles.

"Labeled substance" or "conjugate" refers to a substance that includes a detectable label attached to a specific binding member. The attachment may be covalent or non-covalent binding and may include nucleic acid hybridization. The label allows the labeled substance to produce a detectable signal that is directly or indirectly related to the amount of analyte in a test sample. The specific binding member component of the labeled substance is selected to bind directly or indirectly to the analyte.

"Specific binding member" refers to a member of a specific binding pair (e.g., two different molecules wherein one of the molecules specifically binds to the second molecule through chemical or physical means). If the specific binding member is an immunoreactant it can be, for example, an antibody, antigen, hapten, or complex thereof, and if an antibody is used, it can be a monoclonal or polyclonal antibody, a recombinant protein or antibody, a chimeric antibody, a mixture(s) or fragment(s) thereof, as well as a mixture of an antibody and other specific binding members. Specific examples of specific binding members include biotin and avidin, an antibody and its corresponding antigen (both having no relation to a sample to be assayed), a single stranded nucleic acid and its complement, and the like.

A "test strip" or "LFA" can include one or more bibulous or non-bibulous materials. If a test strip includes more than one material, the one or more materials are preferably in fluid communication. One material of a test strip may be overlaid on another material of the test strip, such as for example, filter paper overlaid on nitrocellulose. Additionally or alternatively, a test strip may include a region including one or more materials (e.g., media) followed by a region including one or more different materials. In this case, the regions are in fluid communication and may or may not partially overlap one another. Suitable materials for test strips include, but are not limited to, materials derived from cellulose, such as filter paper, chromatographic paper, nitrocellulose, and cellulose acetate, as well as materials made of glass fibers, nylon, dacron, polyvinyl chloride (PVC), polyacrylamide, cross-linked dextran, agarose, polyacrylate, ceramic materials, and the like. The material or materials of the test strip may optionally be treated to modify their capillary flow characteristics or the characteristics of the applied sample. For example, the sample application region of the test strip may be treated with buffers to correct the pH or specific gravity of an applied urine sample, to ensure optimal test conditions.

The material or materials can be a single structure such as a sheet cut into strips or it can be several strips or particulate material bound to a support or solid surface such as found, for example, in thin-layer chromatography and may have an absorbent pad either as an integral part or in liquid contact. The material can also be a sheet having lanes thereon, capable of spotting to induce lane formation, wherein a separate assay can be conducted in each lane. The material can have a rectangular, circular, oval, triagonal or other shape provided that there is at least one direction of traversal of a test solution by capillary migration. Other directions of traversal may occur such as in an oval or circular piece contacted in the center with the test solution. However, the main consideration is that there be at least one direction of flow to a predetermined site. In the following discussion test strips will be described by way of illustration and not limitation.

The support for the test strip, where a support is desired or necessary, will normally be water insoluble, frequently non-porous and rigid but may be elastic, usually hydrophobic, and porous and usually will be of the same length and width as the strip but may be larger or smaller. The support material can be transparent, and, when a test device disclosed herein is assembled, a transparent support material can be on the side of the test strip that can be viewed by the user, such that the transparent support material forms a protective layer over the test strip where it may be exposed to the external environment, such as by an aperture in the front of a test device. A wide variety of non-mobilizable and non-mobilizable materials, both natural and synthetic, and combinations thereof, may be employed provided only that the support does not interfere with the capillary action of the material or materials, or non-specifically bind assay components, or interfere with the signal producing system. Illustrative polymers include polyethylene, polypropylene, poly (4-methylbutene), polystyrene, polymethacrylate, poly (ethylene terephthalate), nylon, poly (vinyl butyrate), glass, ceramics, metals, and the like. Elastic supports may be made of polyurethane, neoprene, latex, silicone rubber and the like. Throughout this description, LFAs are described with the understanding that description of the LFAs applies to other types of test strips.

In some conventional LFAs, the test results appear as faint color changes that result in an increase in user error when reading. For example, although an LFA may output a color corresponding to a positive result, if the color is faint and/or the lighting conditions are poor, a user may interpret the test as negative. Examples disclosed herein generates an enhanced LFA that is machine readable that reduces and/or otherwise eliminates false positives and/or false negatives due to human error.

Because resources may be limited in particular regions of the world, dedicated LFA readers may be too expensive for use in such regions. Accordingly, examples disclosed herein provide an enhanced LFA that can be read using a smartphone application, as opposed to a dedicated reader. Although there are some smartphone applications that may be capable of reading the result of an LFA-based test, such smartphone applications are not robust due to alignment and/or environment light issues. For example, if the relative position of a smartphone camera to the LFA test is not within a threshold range, the smartphone application will not be able to determine valid results. In another example, if there is too much light reflection and/or low light conditions when the smartphone captures an image, the smartphone application will not be able to determine valid results.

Examples disclosed herein generate the enhanced LFA to address the alignment and environment light issues that correspond to inaccurate results of the conventional LFA readers. To reduce and/or otherwise eliminate alignment issues, examples disclosed herein include a calibration image and/or pattern on the LFA housing. In this manner, a sensor in the smartphone can scan the calibration image and/or pattern and calibrate itself to read the results of the LFA testing zones regardless of the position of the LFA with respect to the sensor of the smartphone. Additionally, regulations (e.g., medical device regulation (MDR) and/or in vitro diagnostic device regulations (IVDR)) and/or regulating entities may require that smartphones meet particular validation requirements (e.g., optical system specifications), examples disclosed herein can utilize the calibration image and/or pattern to verify whether a sensor of the smartphone meets the validation requirements based on an image of the calibration pattern captured by the sensor.

To reduce and/or otherwise eliminate environment light issues, examples disclosed herein add redundancy to the test zones (e.g., targets) of the LFA. For example, instead of having one test line or test zone corresponding to a particular target analyte, examples disclosed herein have multiple test zones corresponding to the same target analyte. Accordingly, if a sample includes a target analyte, most multiple test zones present evidence of the presence of the target analyte (e.g., by presenting an optical signal such as, for example, a color change, a presence or absence of fluorescence, a presence or absence of one or more letters, numbers, and/or symbols). Examples disclosed herein determine if the test is positive or negative by determining how many of the multiple test zones present a positive test result. For example, if there are five test zones that correspond to a target analyte, and a smartphone application determines that four of the five zones present a positive result (e.g., where the fifth zone presents a negative result due to poor lighting, a reflection, a smudge on the sensor, etc.), the smartphone application will determine that the test was positive for the particular analyte. In this manner, if a particular region is too faint, the light is poor, and/or there is a reflection over the particular region, affecting the result of one test zone, there are other test zones that can compensate for any reading error of the one test zone. Accordingly, the higher redundancy increases the reliability of the results from the smartphone application.

At times, fraudulent entities may target LFAs for counterfeiting and/or forgery. For example, a counterfeiter may obtain a particular LFA and generate counterfeit LFAs to distribute for profit. Examples disclosed herein integrate security features in the enhanced LFA to reduce counterfeiting. Examples disclosed herein segment L number of test lines of an LFA corresponding to N target analytes into M parts distributed to the L lines to generate L*M target zones in a two dimensional grid structure. A two dimensional grid structure includes at least two spatially distinct rows and at least two spatially distinct columns. Examples disclosed herein randomly assign N target analytes to one or more (e.g., more for redundancy) of the L*M target zones in a test grid pattern. Additionally, examples disclosed herein leave one or more of the target zones blank and dedicate one or more of the target zones for one or more control zones (e.g., that capture excess probes resulting from the sample flowing through the testing zones). A blank zone (BZ) is a zone that i) includes antibodies or antigens which do not bind specifically to any target of the sample of ii) does not include immobilized antigens or antibodies. Thus, a blank zone will not change colors regardless of whether there are target analytes in the sample. A control zone (CZ) is a zone that changes color when the LFA test is ready to be read. In this manner, the total number of random distributions of test zones, control zones, and blank zones is equal to (M!)^{N}, when L = M or, more generally, (N!/(L-M)!)^{N}. Examples disclosed herein use all, or a portion of, the total number of random distributions to generate different test grid patterns for different enhanced LFAs. In this manner, when the smartphone applications that read the LFAs identify that a particular test grid pattern is being used more than the others, examples disclosed herein can flag the pattern as having been reproduced by counterfeiters and used to make counterfeit LFAs. Action can be taken to eliminate the counterfeiting including, for example, the smartphone application providing a message that the LFA is not authentic or genuine and/or the smartphone application blocking presentation of the results of the test(s) conducted on the LFA.

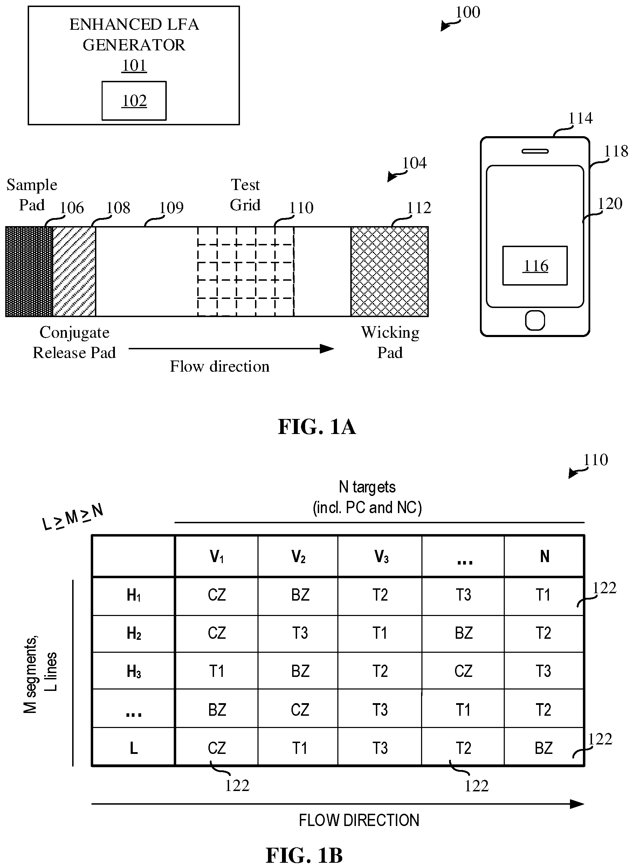

FIG. 1A is an example environment 100 including an example enhanced LFA generator 101 to generate an example enhanced LFA chip, strip, or device 104 (illustrated in an overhead view). The example enhanced LFA generator 101 includes an example test grid generator 102. The example enhanced LFA device 104 includes an example sample pad 106, an example conjugate release pad 108, an example porous media 109, an example test grid 110, and an example wicking pad 112. The environment 100 of FIG. 1 further includes an example reader 114 to determine the test results of a sample being applied to the example enhanced LFA device 104. The example reader 114 includes an example enhanced LFA reader application 116, an example sensor 118, and an example user interface 120.

The example enhanced LFA generator 101 of FIG. 1A generates the example enhanced LFA device 104. For example, the enhanced LFA generator 101 generates the enhanced LFA device 104 to include the example sample pad 106, the example conjugate release pad 108, the example porous media 109, the example test grid 110, and the example wicking pad 112 included in a housing structure (not shown). Additionally, the enhanced LFA generator 101 may include a code (e.g., an identifier (e.g., a data matrix code (DMC)), a pattern or image corresponding to the identifier, and/or a calibration pattern and/or image on or within the housing structure of the enhanced LFA device 104, a barcode, a QR code, etc.) and/or a component that may transmit the code (e.g., a radio frequency transmitter (RFID) transmitter, a near field communication (NFC) transmitter, etc.). A DMC may be a number or code that reflects a production date, an expiry date, a unique identification production identifier (UDI-PI), etc.

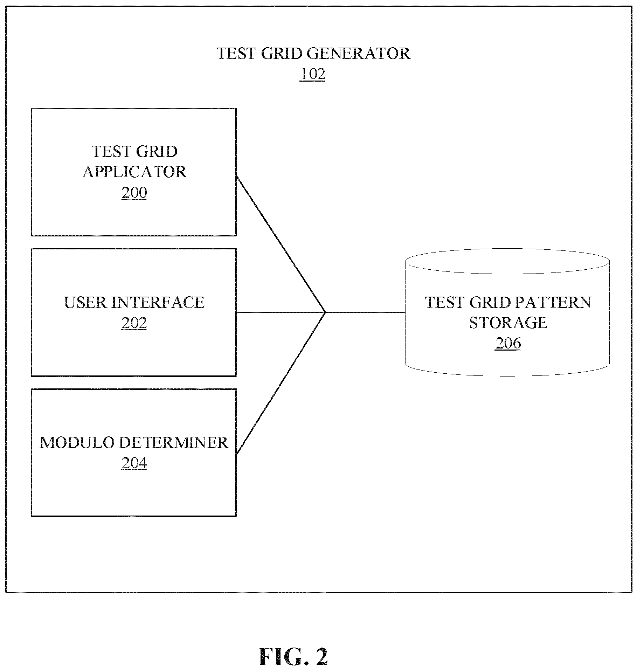

The example enhanced LFA generator 101 of FIG. 1A includes the example test grid generator 102 to generate the example test grid 110 and/or the identifier (e.g., the DMC), the pattern or image corresponding to the identifier, and/or the calibration pattern and/or image on the housing structure of the enhanced LFA device 104. As further disclosed herein, the example test grid generator 102 generates the test grid 110 to include multiple testing zones corresponding to one or more controls, one or more target analytes, and one or more blanks in a two dimensional grid. A test zone corresponding to a target analyte is included in multiple zones of the test grid, thereby providing redundancy to increase reliability and/or robustness of the LFA results. The example test grid generator 102 incorporates a test grid pattern into the test grid 110. The test grid pattern corresponds to an identifier (e.g., DMC) of the enhanced LFA device 104. For example, the test grid generator 102 may compute a function (e.g., a modulo function, a checksum, etc.) of the DMC resulting in a number that corresponds to a test grid pattern. Accordingly, the test grid generator 102 structures the test grid 110 based on the resulting number. For example, if the enhanced LFA generator 101 generates 100,000 LFAs with 1,000 different test grid patterns, when the enhanced LFA generator 101 generates the 76,986^{th} LFA, the test grid generator 102 selects the test grid pattern corresponding to number 986 (e.g., 76,986 modulo 1000 = 986). In this matter, when the enhanced LFA reader application 116 interacts with the enhanced LFA device 104, the enhanced LFA reader application 116 can determine which test grid pattern to use to analyze the test grid 110 based on the same function (e.g., modulo, checksum, etc.). The example test grid generator 102 is further disclosed below in conjunction with FIG. 2.

The example enhanced LFA device 104 of FIG. 1A is a device that includes the sample pad 106 (e.g., a sample pad, a sample region, a sample area, a sample zone, etc.). For example, the LFA device 104 may be a porous membrane device, a porous media device, a fluid transporting media device, a test strip device, a lateral flow test strip device, and/or any fluid sample device. The sample pad 106 is structured to act as a sponge to hold a sample of fluid applied to the sample pad 106. In some examples, the sample pad 106 includes buffer components (e.g., salts, surfactants, etc.) to ensure that target analytes that may be in the sample are capable of binding with components of the conjugate release pad 108. When the sample pad 106 is soaked, the fluid held in the sample pad 106 flows to the conjugate release pad 108. The conjugate pad 108 includes a labeled substance or conjugate configured to bind a target analyte. For example, the conjugate release pad 108 includes conjugates or probes (e.g., gold, latex, fluorophore, etc.) labeled with detectable labels, tags, linkers, antibodies, antigens, etc. specific to one or more target analyte. The target analyte is a component that corresponds to a particular condition or disease. Accordingly, presence of the target analyte in the sample corresponds to presence of the corresponding condition and/or disease in the patient who provided the sample. If the sample includes the one or more of the target analytes, the conjugates and/or probes labelled with the detectable labels, tags, linkers, antibodies, antigens, etc. will attach to the corresponding target analytes. The sample (e.g., including the probes if corresponding target analytes are present in the sample) continues to flow through the example porous media 109 (e.g., a membrane, a paper, and/or other compartment-free or compartmentless substrate) of the enhanced LFA device 104 toward the wicking pad 112.

While the sample flows across the media 109 of FIG. 1A, the sample will flow across the test grid 110. The test grid 110 includes test zones and/or control zones. The test zones include specific immobilized antibodies or antigens that react with the corresponding analytes attached with the probes and conjugates. Accordingly, when the target analyte corresponding to a particular test zone is present in the sample, the target analyte will react with the antibodies resulting in an optical signal such as, for example, a change in color (e.g., from white to red). In some examples, the test grid 110 includes control zones that change color when excess conjugates flow past the control zones. Excess conjugates flowing to the control zones indicate that sufficient conjugate has flowed through the test zones. Thus, the optical signal changes in the control zones identify when it is appropriate to read the test results (e.g., when the test is complete). In other examples, the media 109 may include a control line and/or zone located between the test grid 110 and the wicking pad 112. Because the test grid 110 corresponds to one of a plurality of test grid patterns, if the test grid patterns are not shared with the patients and/or users, it is not possible to determine the results of the LFA test by looking at the results. For example, the results will appear random to the patient and/or user because the patient and/or user will not know which zone(s) correspond(s) to test zone(s), which zone(s) correspond(s) to a control zone(s), and which zone(s) correspond(s) to blank zone(s). Accordingly, in such an example, the test grid 110 can only be read by the example enhanced LFA reader application 116, thereby reducing, or otherwise eliminating, user error caused by faint optical results and/or poor lighting conditions. An example implementation of the test grid 110 is further disclosed below in conjunction with FIG. 1B.

The example wicking pad 112 is an absorbent material that wicks the liquid through the LFA. In some examples, the wicking pad 112 includes cellulose filters. The wicking pad 112 prevents backflow of the liquid. Also, in some examples, the wicking pad 112 acts as a waste container.

**In** some examples, silver amplification may be applied to the example enhanced LFA 104 to increase the visibility of the results. In such examples, the enhanced LFA generator 101 may apply an autocatalytic silver enhancement (e.g., silver ion and reducing agent, such as hydroquinone, aminophenols, ascorbic acid) to the test grid 110 so that the silver reacts with the conjugate and amplifies in size, thereby generating a stronger visual signal. The enhanced LFA generator 101 may structure the enhanced LFA 104 to include an inlet so that the silver enhancement may be applied to the example test grid 110 during or after the sample is applied.

The example reader 114 of FIG. 1A is a smartphone that includes the enhanced LFA reader application 116, the example sensor 118, and the example user interface 120. Alternatively, the example reader 114 may be a tablet, personal digital assistant, a laptop, a standalone LFA reader device, and/or any other processing device that includes, or is otherwise in communication with, the example enhanced LFA reader application 116, the example sensor 118, and/or the example user interface 120. The example enhanced LFA reader application 116 is an application that can be installed, downloaded, or coded within the example reader 114. In some examples, the example enhanced LFA reader application 116 can be supplied via a near field communication tag or Bluetooth device.

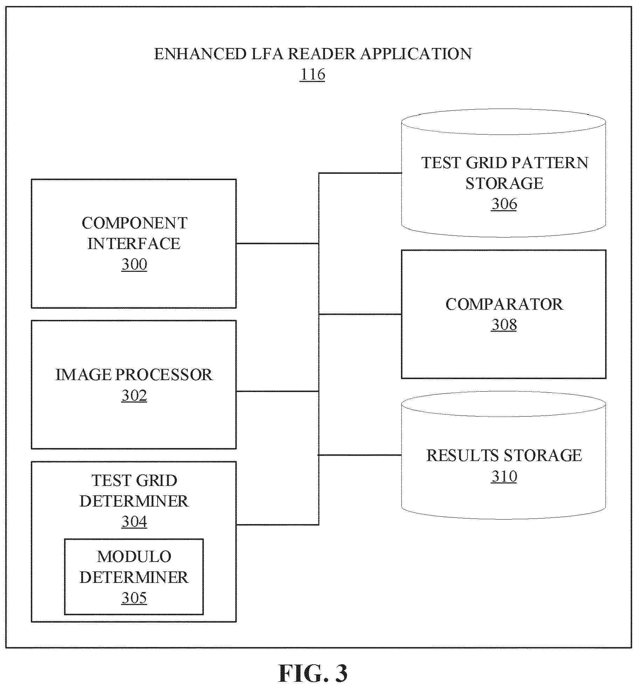

The example enhanced LFA reader application 116 of FIG. 1A identifies the results of an LFA-based test by interacting with the example LFA structure. The enhanced LFA reader application 116 controls the components of the reader 114 to obtain identification information (e.g., a DMC or test grid pattern identifier) corresponding to the enhanced LFA device 104. The enhanced LFA reader application 116 obtains identification information from the enhanced LFA device 104 to determine which test grid pattern is applied to the example test grid 110. For example, the enhanced LFA reader application 116 may obtain an LFA identifier and/or a DMC from the user interface 120 (e.g., by prompting the user to enter the information) or from the sensor 118 (e.g., by processing an image captured by the sensor 118 that corresponds to the information). In some example, the enhanced LFA reader application 116 performs an operation (e.g., a modulo operation, a checksum, etc.) on the identification information to determine which test grid pattern is implemented on the enhanced LFA device 104, as further described above. Once the test grid pattern of the test grid 110 is determined, the enhanced LFA reader application 116 determines the results of the test by determining the optical signal (e.g., color) of the test zones corresponding to the test grid pattern. Once the test results are determined, the enhanced LFA reader application 116 displays the results using the example user interface 120, stores the results in a local database, and/or transmits the results to a monitoring entity for monitoring and/or statistical analysis. In this manner, the manufacturer or other party can process the results, perform diagnostics, and/or identify if a particular test grid pattern may be counterfeited (e.g., if more than a threshold number of results from an LFA with the test grid pattern has been determined). In some examples, the LFA reader application 116 transmits raw data. Additionally or alternatively, in some examples, the LFA reader application 116 transmits processed data or data resulting from one or more levels of analysis. The example enhanced LFA reader application 116 is further disclosed below in conjunction with FIG. 3.

FIG. 1B illustrates an implementation of the example test grid 110 of FIG 1A. The example test grid 110 includes twenty-five examples zones 122 (e.g., five rows (A-E) and five columns (1-5)). Alternatively, any number of zones in combination of rows and/or columns may be used to implement the example test grid 110.

**In** the example test grid 110 of FIG. 1B, the zones including 'T1' correspond to test zones for a first disease or condition (e.g., that present an optical signal such as, for example, a change in color when a first particular target analyte is present in a sample), the zones including the 'T2' correspond to test zones for a second disease or condition, the zones including the 'T3' correspond to test zones for a third disease or condition, the zones including 'CZ' correspond to a positive control zone (e.g., which present an optical signal such as, for example, a change in color when the test is ready), and the zones including 'BZ' correspond to a blank zone.

The example test grid 110 represents one combination of the zones. Because there are five targets (e.g., three test and two control) segmented into five rows (e.g., parts) across five columns (e.g., lines), the total number of different combinations that can be used in the example test grid 110 is 24,883,200,000 (e.g., (5!)⁵). However, as described above, the number of combinations are different based on how many tests and controls are implemented in the grid, how many rows and/or columns are used for the grid, and the zones of the grids left intentionally blank.

Some LFAs are subject to a depletion effect. A depletion effect is a situation when targets are bound, captured, and/or consumed and the first capture zones in the flow direction show high signal. If a depletion effect is an issue, the example test grid 110 may be structured to include a test zone for a particular analyte in one or more rows but only one column to overcome the depletion effect. Accordingly, in the example test grid 110 of FIG. 1B, each test and control zone occurs in every row but only once in each column. Alternatively, the test and control zones may occur in less numbers of rows.

FIG. 2 is a block diagram of the example test grid generator 102 of FIG. 1A. The example test grid generator 102 includes an example test grid applicator 200, an example user interface 202, an example modulo determiner 204, and an example test grid pattern storage 206.

The example test grid applicator 200 of FIG. 2 determines the structure of the test grid 110 of the enhanced LFA device 104 of FIG. 1A and/or 1B. For example, the test grid applicator 200 may determine how many different target analytes are to be implemented in the enhanced LFA device 104 and determine how many zones to include in the test grid 110 to meet a threshold number of different combinations that can be implemented in the test grids of LFAs. Additionally or alternatively, the example test grid applicator 200 may receive instructions identifying the number of test, controls, and/or structure of the test grid 110 from a user and/or manufacturer via the example user interface 202. Additionally, the test grid applicator 200 may determine how many different combinations of test grids to use when generating the multiple LFAs.

Additionally, the test grid applicator 200 of FIG. 2 may determine how the testing pattern will be indicated to the reader 114. For example, if the test grid applicator 200 is generating 1500 different test grid patterns for newly generated LFAs, the test grid applicator 200 determines how to indicate which test grid pattern is implemented on each LFA so that the reader 114 can determine how to read the test grid 110. In some examples, the test grid applicator 200 selects one of the test grid patterns stored in the test grid pattern storage 206 and applies an identifier (e.g., a number or a code identifying the test pattern) of the test grid structure on the housing structure of the LFA. In some examples, the test grid applicator 200 obtains a modulo or other value of the DMC from the enhanced LFA generator corresponding to the LFA being generated. In such examples, the test grid applicator 200 may select a test grid pattern from the example test grid pattern storage 206 corresponding to the result of the modulo. For example, if the DMC is 5142956 and there are 1250 test grid patterns stored in the test grid pattern storage 206, the example modulo determiner 204 determines the modulo to be 456 (5142953 mod 1250). In such an example, the test grid pattern storage 206 selects the test pattern corresponding to identifier 456 to implement the test grid 110.

The example user interface 202 of FIG. 2 interfaces with a user and/or manufacturer to obtain instructions regarding how to structure the test grid structure. For example, the user interface 202 may receive instructions regarding how many tests to perform, how many test grid patterns to implement, how many columns and/or row to utilize, how many blank spaces to include, how many control zones to include, etc.

The example modulo determiner 204 of FIG. 2 performs a modulo function based on the code (e.g., the DMC code) of the LFA that is currently being generated and the number of test grid patterns in the example test grid pattern storage 206 and/or the number of test grid patterns used per user instructions. For example, if the DMC is 5142956 and there are 1250 test grid patterns stored in the test grid pattern storage 206, the example modulo determiner 204 determines the modulo to be 456 (5142953 mod 1250). In this manner, the example reader 114 of FIG. 1A can perform the same modulo function based on the DMC pattern to identify the corresponding test grid pattern when analyzing the results of an LFA-based test. Additionally or alternatively, the modulo determiner 204 may be another type of function generator to generate a value that corresponds to a test grid pattern.

The example test grid pattern storage 206 of FIG. 2 stores different test grid patterns in conjunction with an identifier. The test grid patterns correspond to which zones of the test grid correspond to different tests, controls, and blanks. In this manner, the example test grid applicator 200 can apply one of the stored test grid patterns to the test grid 110 of the example enhanced LFA device 104 corresponding to an identifier that can be determined at the reader 114.

FIG. 3 is a block diagram of the example enhanced LFA reader application 116 of FIG. 1A. The example test grid generator 102 includes an example component interface 300, an example image processor 302, an example test grid determiner 304, an example test grid determiner 304, an example modulo determiner 305, an example test grid pattern storage 306, an example comparator 308, and an example results storage 310.

The example component interface 300 of FIG. 3 interfaces with the other components of the example reader 114. For example, the component interface 300 may transmit prompts, text, and/or images to the example user interface 120 to display to a user. Additionally, the example component interface 300 receives data entered by the user via the user interface 120. For example, if the user enters the DMC code printed on the housing of the enhanced LFA device 104 into the user interface 120, the example component interface 300 obtains the entered DMC code from the user interface 120. Additionally, the example component interface 300 interfaces with the sensor 118 to obtain images that the sensor 118 captured. In some examples, the component interface 300 interfaces with a transmitter of the reader 114 to transmit results of an LFA-based test reading to an entity that monitors the results. The results may include which tests are positive, which tests are negative, which test grid pattern was used, identification information of the LFA (e.g., DMC), and/or contextual information obtained from the reader 114 and/or a user (e.g., timestamp, location data, patient information and/or demographics, etc.). In this manner, if the entity that monitors the results determines that more than a threshold number of percent of a particular LFA or test grid has been used, the LFA and/or test grid can be flagged as possibly being counterfeit and subsequent actions can be taken to prevent further counterfeiting and/or reliance on test results from a counterfeit device. If a network connection is not available after a test is performed, the component interface 300 may transmit the results after receiving an indication that a network connection is available.

The example image processor 302 of FIG. 3 processes images obtained from the sensor 118 via the example component interface 300. In some examples, the sensor 118 obtains an image of a code (e.g., text, QR code, etc.) printed or otherwise included in and/or on the housing of the enhanced LFA device 104 and/or in and/or on paperwork included with the LFA device 104, and the image processor 302 processes the image to identify the corresponding information. As described above, the housing or interior of the enhanced LFA device 104 may include a DMC code, or other identifier, and/or an image (e.g., QR code) that corresponds to the DMC code or other identifier. Accordingly, if an image is taken of the text and/or code printed on the housing, the image processor 302 can identify the DMC code and/or any other identification information based on the image of the text and/or code. Additionally or alternatively, the enhanced LFA device 104 may include a wireless transmitter (e.g., RFID, NFC, etc.) to transmit the code and/or identifier to the example reader 114.

Additionally, in some examples, the example image processor 302 of FIG. 3 processes an image of a calibration pattern. In this manner, the example image processor 302 can calibrate the sensor 118 according to the obtained image of the calibration pattern. Additionally, the example image processor 302 can process the image of the calibration pattern to determine if the quality of the sensor 118 is sufficient to meet minimum optimal system specifications. If the example image processor 302 determines that the processing of a calibration pattern results in a failure of the optimal system specifications, the image processor 302 prevents processing of an LFA-based test. In some examples, the image processor 302 may attempt to improve the optical system settings by, for example, turning on a light of the reader 114 (e.g., using the component interface 300) and/or prompting the user (e.g., using the user interface 120 via the component interface 300) to turn on a light (e.g., a light of the reader 114 or a light separate from the reader 114) to improve the lighting conditions to retest the optical system specification under better lighting conditions.

Additionally, the example image processor 302 of FIG. 3 processes images of the test grid 110 of the enhanced LFA device 104 to determine which zones of the test grid 110 presented a positive result and which zones presented a negative result. As described above, if a target analyte is present in a sample, corresponding zone(s) of the test grid 110 will present optical signals (e.g., changing such as from white to another color). Accordingly, the image processor 302 processes an image of the test grid 110 to determine which zones correspond to color(s) corresponding to a positive result and which zones correspond to color(s) corresponding to a negative result.

As further disclosed herein, the example test grid determiner 304 may identify which test grid pattern was used to generate the test grid 110. Accordingly, the image processor 302 will know the structure (e.g., the number and/or dimensions of columns and rows) of the test grid 110. In some examples, the structure of the test grid 110 may be preset (e.g., all LFAs having the same test grid structure).

The example test grid determiner 304 of FIG. 3 determines which test grid is used for the example test grid 110 of FIG. 1A. As described above, the user may provide identification information (e.g., using the user interface 120) and/or the identification information may be obtained by the image processor 302 based on an image taken of the identification information. The identification information may be a test grid identifier, a DMC, etc. If the identification information is a test grid identifier, the example test grid determiner 304 identifies the corresponding test grid in the test grid pattern storage 306 based on the test grid identifier. If the identification information is a DMC code, a pattern and/or image that corresponds to a code, and/or any other identification code, the modulo determiner 305 performs a modulo function based on the DMC code of the enhanced LFA device 104 and the number of test grid patterns in the example test grid pattern storage 306 and/or the number of test grid patterns preprogrammed into the enhanced LFA reader application 116. For example, if the DMC is 5142956 and there are 1250 test grid patterns stored in the test grid pattern storage 306, the example modulo determiner 305 determines the modulo to be 456 (5142953 mod 1250). The example modulo determiner 305 operates in a similar fashion as the modulo determiner 305 of FIG. 2. In this manner, the enhanced LFA reader application 116 can determine which test grid pattern was used by the enhanced LFA generator 101 to generate the test grid 110 of the enhanced LFA device 104. Additionally or alternatively, the modulo determiner 305 may be another type of function generate to generate a value that corresponds to a test grid pattern.

The example test grid pattern storage 306 of FIG. 3 stores different test grid patterns in conjunction with an identifier. The test grid patterns correspond to which zones of the test grid correspond to different tests, controls, and blanks. The test grid pattern storage 306 stores the same test grid patterns in conjunction with the same identifiers as the test grid pattern storage 206 of FIG. 2. In this manner, the example test grid determiner 304 can identify which test grid pattern is used in the test grid 110 of the enhanced LFA device 104.

The example comparator 308 of FIG. 3 compares the results of multiple zone(s) that correspond to the same target analyte to determine whether the test result is positive or negative. For example, if there are six zones on the test grid 110 that correspond to HIV, the comparator 308 compares the number of positive results and/or the number of negative results of the six zones to one or more thresholds. In such an examples, the comparator 308 may determine that the test resulted positive for HIV if four or more zones presented a positive result, that the test resulted negative for HIV if four or more zones presented a negative result, and that the test resulted indeterminate for HIV if three zones were presented a positive result and three zones presented a negative result. In some examples, the threshold number of zones may be based on the type of analyte to be detected, industry and/or government regulations, user preferences, manufacturer preferences or recommendations, and/or a combination of criteria.

The example results storage 310 of FIG. 3 stores the results of an LFA test (e.g., which tests presented positive results, negative results, or indeterminate results, and/or any corresponding information) in conjunction with any identification information (e.g., DMC, test grid identifiers, etc.). In some examples, a user can enter patient information via the user interface 120. In such examples, some or all of the patient information may be added to the result information (e.g., the results storage 310 stores a record of the test result in conjunction with the patient information). Additionally or alternatively, the example component interface 300 may gather contextual information when the test is performed that may be stored in the example results storage 310 in conjunction with the results (e.g., location information, time of day information, etc.).

While an example manner of implementing the example test grid generator 103 of FIG. 1A is illustrated in FIG. 2 and an example manner of implementing the example enhanced LFA reader application 116 of FIG. 1A is illustrated in FIG. 3, one or more of the elements, processes and/or devices illustrated in FIGS. 2 and/or 3 may be combined, divided, re-arranged, omitted, eliminated and/or implemented in any other way. Further, the example test grid applicator 200, the example user interface 202, the example modulo determiner 204, the example test grid pattern storage 206, and/or, more generally, the example test grid generator 102 of FIG. 2 and the example component interface 300, the example image processor 302, the example test grid determiner 304, the example modulo determiner 305, the example test grid pattern storage 206, the example comparator 308, the example results storage 310, and/or, more generally, the example enhanced LFA reader application 116 may be implemented by hardware, software, firmware and/or any combination of hardware, software and/or firmware. Thus, for example, any of the example test grid applicator 200, the example user interface 202, the example modulo determiner 204, the example test grid pattern storage 206, and/or, more generally, the example test grid generator 102 of FIG. 2 and the example component interface 300, the example image processor 302, the example test grid determiner 304, the example modulo determiner 305, the example test grid pattern storage 206, the example comparator 308, the example results storage 310, and/or, more generally, the example enhanced LFA reader application 116 could be implemented by one or more analog or digital circuit(s), logic circuits, programmable processor(s), programmable controller(s), graphics processing unit(s) (GPU(s)), digital signal processor(s) (DSP(s)), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example test grid applicator 200, the example user interface 202, the example modulo determiner 204, the example test grid pattern storage 206, and/or, more generally, the example test grid generator 102 of FIG. 2 and the example component interface 300, the example image processor 302, the example test grid determiner 304, the example modulo determiner 305, the example test grid pattern storage 206, the example comparator 308, the example results storage 310, and/or, more generally, the example enhanced LFA reader application 116 is/are hereby expressly defined to include a non-transitory computer readable storage device or storage disk such as a memory, a digital versatile disk (DVD), a compact disk (CD), a Blu-ray disk, etc. including the software and/or firmware. Further still, the example test grid generator 102 of FIG. 2 and the example enhanced LFA reader application 116 of FIG. 3 may include one or more elements, processes and/or devices in addition to, or instead of, those illustrated in FIGS. 2 and 3, and/or may include more than one of any or all of the illustrated elements, processes and devices. As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

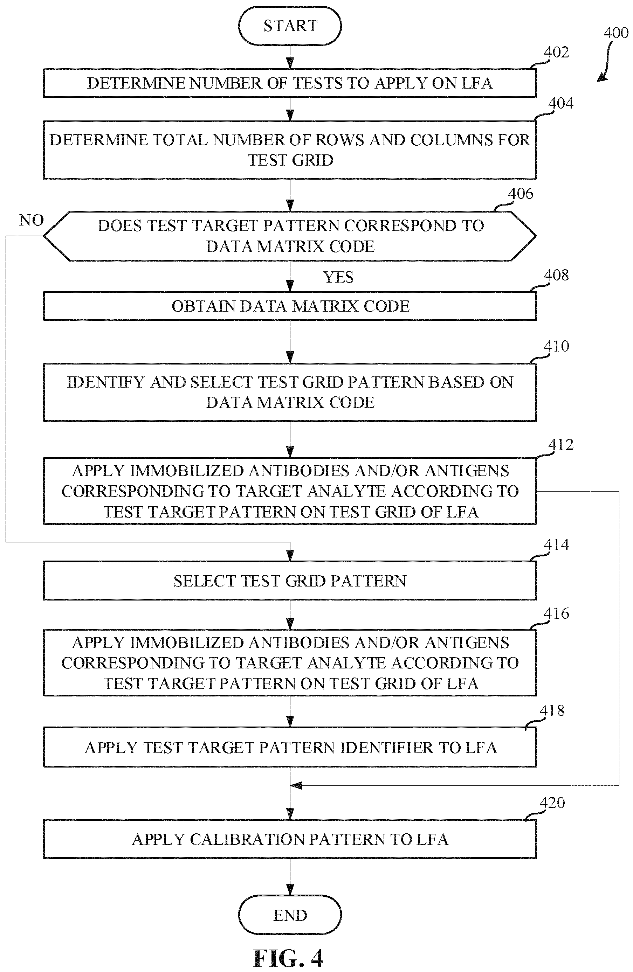

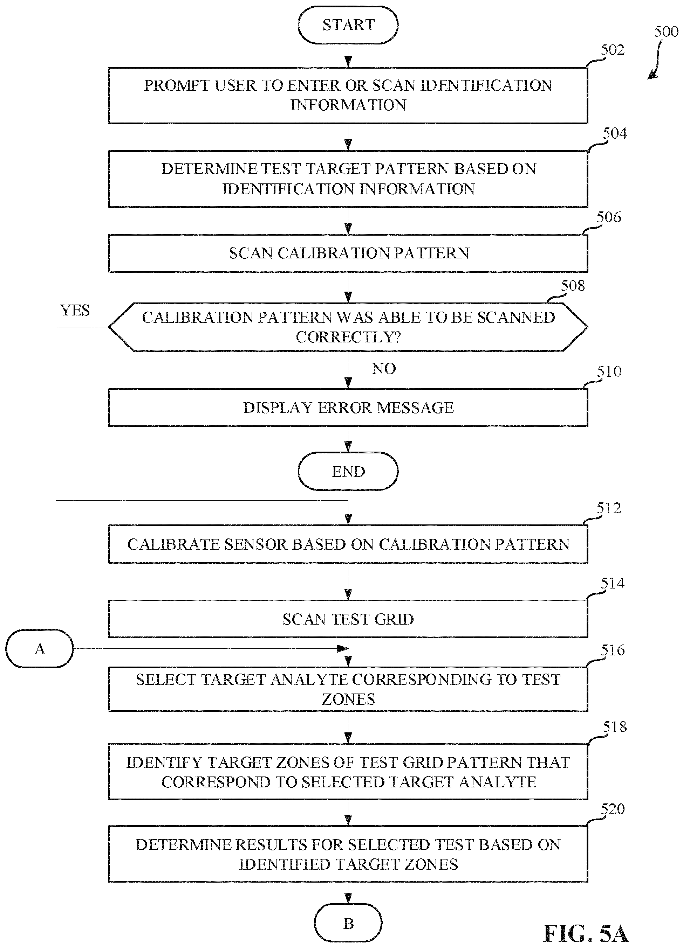

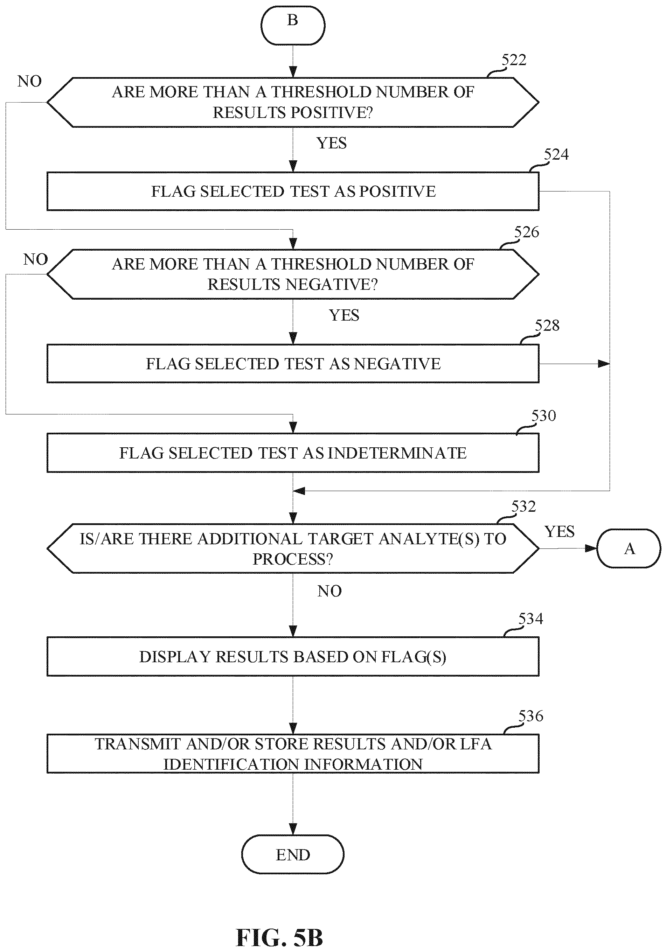

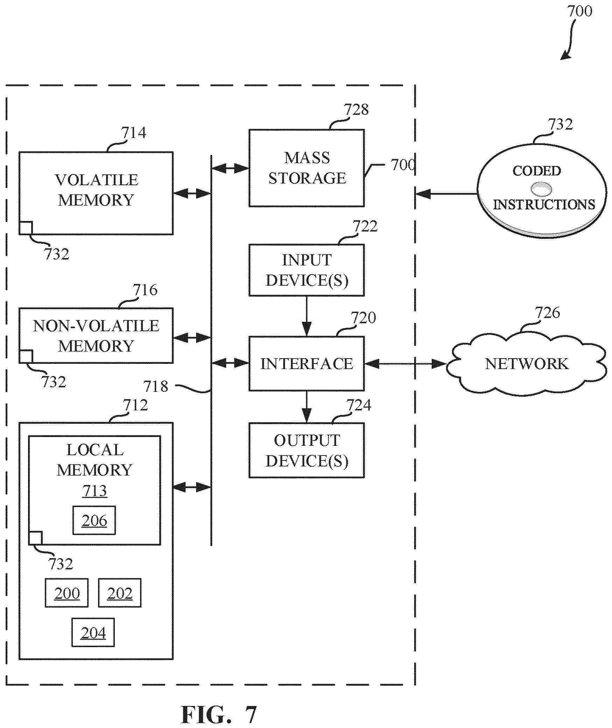

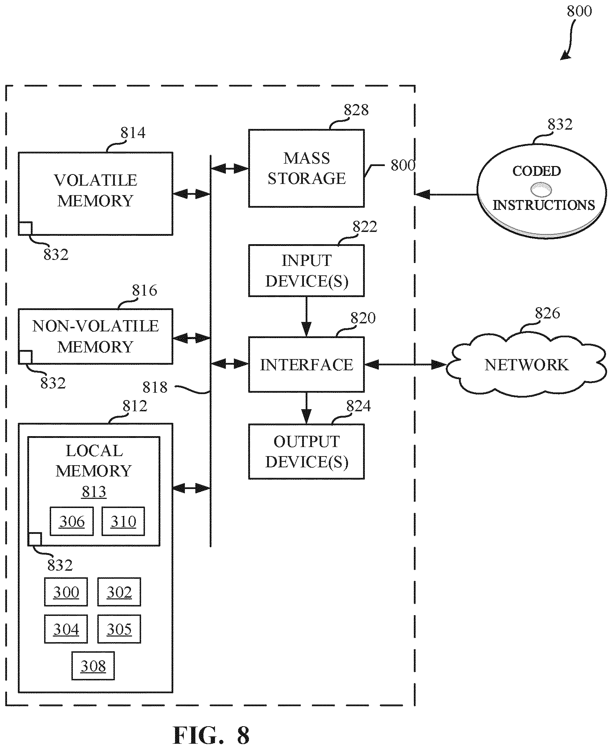

Flowcharts representative of example hardware logic, machine readable instructions, hardware implemented state machines, and/or any combination thereof for implementing the test grid generator 102 and/or the enhanced LFA reader application 116 of FIGS. 2 and/or 3 are shown in FIGS. 4, 5A, and 5B. The machine readable instructions may be one or more executable programs or portion(s) of an executable program for execution by a computer processor such as the processor 712, 812 shown in the example processor platform 700, 800 discussed below in connection with FIGS. 7 and/or 8. The program may be embodied in software stored on a non-transitory computer readable storage medium such as a CD-ROM, a floppy disk, a hard drive, a DVD, a Blu-ray disk, or a memory associated with the processor 712, 812 but the entire program and/or parts thereof could alternatively be executed by a device other than the processor 712, 812 and/or embodied in firmware or dedicated hardware. Further, although the example program is described with reference to the flowcharts illustrated in FIGS. 4, 5A, and/or 5B many other methods of implementing the example test grid generator 102 and/or the example enhanced LFA reader application 116 may alternatively be used. For example, the order of execution of the blocks may be changed, and/or some of the blocks described may be changed, eliminated, or combined. Additionally or alternatively, any or all of the blocks may be implemented by one or more hardware circuits (e.g., discrete and/or integrated analog and/or digital circuitry, an FPGA, an ASIC, a comparator, an operational-amplifier (op-amp), a logic circuit, etc.) structured to perform the corresponding operation without executing software or firmware.

The machine readable instructions described herein may be stored in one or more of a compressed format, an encrypted format, a fragmented format, a compiled format, an executable format, a packaged format, etc. Machine readable instructions as described herein may be stored as data (e.g., portions of instructions, code, representations of code, etc.) that may be utilized to create, manufacture, and/or produce machine executable instructions. For example, the machine readable instructions may be fragmented and stored on one or more storage devices and/or computing devices (e.g., servers). The machine readable instructions may require one or more of installation, modification, adaptation, updating, combining, supplementing, configuring, decryption, decompression, unpacking, distribution, reassignment, compilation, etc. in order to make them directly readable, interpretable, and/or executable by a computing device and/or other machine. For example, the machine readable instructions may be stored in multiple parts, which are individually compressed, encrypted, and stored on separate computing devices, wherein the parts when decrypted, decompressed, and combined form a set of executable instructions that implement a program such as that described herein.

In another example, the machine readable instructions may be stored in a state in which they may be read by a computer, but require addition of a library (e.g., a dynamic link library (DLL)), a software development kit (SDK), an application programming interface (API), etc. in order to execute the instructions on a particular computing device or other device. In another example, the machine readable instructions may need to be configured (e.g., settings stored, data input, network addresses recorded, etc.) before the machine readable instructions and/or the corresponding program(s) can be executed in whole or in part. Thus, the disclosed machine readable instructions and/or corresponding program(s) are intended to encompass such machine readable instructions and/or program(s) regardless of the particular format or state of the machine readable instructions and/or program(s) when stored or otherwise at rest or in transit.

The machine readable instructions described herein can be represented by any past, present, or future instruction language, scripting language, programming language, etc. For example, the machine readable instructions may be represented using any of the following languages: C, C++, Java, C#, Perl, Python, JavaScript, HyperText Markup Language (HTML), Structured Query Language (SQL), Swift, etc.

As mentioned above, the example processes of FIGS. 4, 5A, and/or 5B may be implemented using executable instructions (e.g., computer and/or machine readable instructions) stored on a non-transitory computer and/or machine readable medium such as a hard disk drive, a flash memory, a read-only memory, a compact disk, a digital versatile disk, a cache, a random-access memory and/or any other storage device or storage disk in which information is stored for any duration (e.g., for extended time periods, permanently, for brief instances, for temporarily buffering, and/or for caching of the information). As used herein, the term non-transitory computer readable medium is expressly defined to include any type of computer readable storage device and/or storage disk and to exclude propagating signals and to exclude transmission media.

"Including" and "comprising" (and all forms and tenses thereof) are used herein to be open ended terms. Thus, whenever a claim employs any form of "include" or "comprise" (e.g., comprises, includes, comprising, including, having, etc.) as a preamble or within a claim recitation of any kind, it is to be understood that additional elements, terms, etc. may be present without falling outside the scope of the corresponding claim or recitation. As used herein, when the phrase "at least" is used as the transition term in, for example, a preamble of a claim, it is open-ended in the same manner as the term "comprising" and "including" are open ended. The term "and/or" when used, for example, in a form such as A, B, and/or C refers to any combination or subset of A, B, C such as (1) A alone, (2) B alone, (3) C alone, (4) A with B, (5) A with C, (6) B with C, and (7) A with B and with C. As used herein in the context of describing structures, components, items, objects and/or things, the phrase "at least one of A and B" is intended to refer to implementations including any of (1) at least one A, (2) at least one B, and (3) at least one A and at least one B. Similarly, as used herein in the context of describing structures, components, items, objects and/or things, the phrase "at least one of A or B" is intended to refer to implementations including any of (1) at least one A, (2) at least one B, and (3) at least one A and at least one B. As used herein in the context of describing the performance or execution of processes, instructions, actions, activities and/or steps, the phrase "at least one of A and B" is intended to refer to implementations including any of (1) at least one A, (2) at least one B, and (3) at least one A and at least one B. Similarly, as used herein in the context of describing the performance or execution of processes, instructions, actions, activities and/or steps, the phrase "at least one of A or B" is intended to refer to implementations including any of (1) at least one A, (2) at least one B, and (3) at least one A and at least one B.

As used herein, singular references (e.g., "a", "an", "first", "second", etc.) do not exclude a plurality. The term "a" or "an" entity, as used herein, refers to one or more of that entity. The terms "a" (or "an"), "one or more", and "at least one" can be used interchangeably herein. Furthermore, although individually listed, a plurality of means, elements or method actions may be implemented by, e.g., a single unit or processor. Additionally, although individual features may be included in different examples or claims, these may possibly be combined, and the inclusion in different examples or claims does not imply that a combination of features is not feasible and/or advantageous.

FIG. 4 illustrates an example flowchart representative of machine readable instructions 400 that may be executed to implement the example testing grid generator 102 of FIG. 2 to generate the test grid 110 of the example enhanced LFA device 104 of FIG. 1A. Although the instructions 400 of FIG. 4 are described in conjunction with the example test grid 110 of the example enhanced LFA device 104 of FIG. 1A, the example instructions 400 may be described and/or implemented in conjunction with any type of test grid and/or LFA of any structure.

At block 402, the example test grid applicator 200 determines the number of tests to apply on the LFA device 104. For example, the enhanced LFA generator 101 may provide instructions regarding how many tests corresponding to target analytes should be applied on the test grid 110. Additionally or alternatively, a user or administrator may provide the number of tests via the example user interface 202.

At block 404, the example test grid applicator 200 determines a total number of row columns for the test grid. In some examples, the test grid applicator 200 may determine the total number of rows and columns for the test grid based on instructions (e.g., from the enhanced LFA generator 101 and/or from a user via the user interface 202). In some examples, the number of rows and/or columns is preset. In some examples, the number of rows and/or columns corresponds to the number of tests (e.g., if there are three tests, there are three or more rows and/or columns). In some examples, the number of rows and/or columns is more than a preselected minimum amount (e.g., that may be set by a user, administrator, and/or regulations).

At block 406, the example test grid applicator 200 determines if the test grid pattern corresponds to a DMC. For example, the enhanced LFA generator 101 may generate a DMC including information corresponding to the currently generated LFA. The enhanced LFA generator 101 may instruct the test grid applicator 200 to select a test grid from the test grid pattern storage 206 randomly or based on the DMC. If the example test grid applicator 200 determines that the test grid pattern does not correspond to a DMC (block 406: NO), control continues to block 414, as further described below. If the example test grid applicator 200 determines that the test grid pattern does correspond to a DMC (block 406: YES), the example test grid applicator 200 obtains the DMC from the enhanced LFA generator 101 (block 408).

At block 410, the example modulo determiner 204 identifies the test grid pattern corresponding to the DMC and selects the test grid pattern from the example test grid pattern storage 206. As disclosed above in conjunction with FIG. 2, the modulo determiner 204 may perform a modulo operation or another type of operation (e.g., checksum) on the DMC to generate a number that corresponds to a test grid pattern stored in the example test grid pattern storage 206. At block 412, the example test grid applicator 200 applies the immobilized antibodies and/or antigens corresponding to a target analyte to a zone of the test grid 110 of the enhanced LFA device 104 according to the selected test grid pattern. In some examples, the test grid applicator 200 additionally applies immobilized antibodies and/or antigens corresponding to control zones to attach to excess antibodies and/or control antibodies.

If the example test grid applicator 200 determines that the test grid pattern does not correspond to a DMC (block 406: NO), the example test grid applicator 200 selects a test grid pattern from the example test grid pattern storage 206 (block 414). The test grid applicator 200 may select a test grid pattern randomly, sequentially, and/or based on any other pattern. At block 416, the example test grid applicator 200 applies the immobilized antibodies and/or antigens corresponding to a target analyte to a zone of the test grid 110 of the enhanced LFA device 104 according to the selected test grid pattern. In some examples, the test grid applicator 200 additionally applies immobilized antibodies and/or antigens corresponding to control zones to attach to excess antibodies and/or control antibodies.

At block 418, the example test grid applicator 200 applies a test target pattern identifier (e.g., text, a QR code, etc.) to and/or within the housing of the enhanced LFA device 104, and/or to another component and/or paper associated with the LFA device 104. In this manner, the enhanced LFA reader application 114 can identify which test grid is used on the enhanced LFA device 104 for test reading purposes. At block 420, the example test grid applicator 200 applies a calibration pattern to the LFA. The calibration pattern allows the enhanced LFA reader application 116 to determine whether the sensor 118 of the reader 114 has sufficient optical system specification and/or to calibrate the sensor 118 for an LFA-based reading.

FIGS. 5A and 5B illustrate an example flowchart representative of machine readable instructions 500 that may be executed to implement the example enhanced LFA reader application 116 of FIG. 3 to read test results of the example enhanced LFA device 104 of FIG. 1A. Although the instructions 500 of FIG. 5 are described in conjunction with the example enhanced LFA device 104 of FIG. 1A, the example instructions 500 may be described and/or implemented in conjunction with type of LFA of any structure.

At block 502, the example component interface 300 prompts a user (e.g., via the user interface 120) to enter (e.g., using the user interface 120) or scan (e.g., using the sensor 118) identification information (e.g., a DMC, test grid identification information, etc.) from the enhanced LFA device 104. In some examples, if the identification information is scanned (e.g., by obtaining an image of the identification information), the example image processor 302 determines the identification information by processing the image. Additionally or alternatively, the identification information may be a code that corresponds to a test grid pattern. In some examples, the component interface 300 may obtain a code and/or identification information from a receiver (e.g., when the code and/or identification information is transmitted via RFID, NFC, etc.). The identification information is passed to the example test grid determiner 304.

At block 504, the example test grid determiner 304 determines the test grid pattern based on the identification information. For example, if the test grid information is an identifier of a test grid, the example test grid determiner 304 obtains the test grid corresponding to the identifier from the test grid pattern storage 306. If the test grid information is a DMC, the example modulo determiner 305 performs a modulo function, or other function, on the DMC to determine the test grid identifier, and the test grid determiner 304 obtains the test grid corresponding to the identifier from the test grid pattern storage 306.

At block 506, the example component interface 300 instructs the sensor 118 to scan the calibration pattern on the housing of the enhanced LFA device 104. The calibration pattern may be a pattern that can only be read and/or processed if the optical system specifications of the reader 114 are above minimum requirements. At block 508, the example image processor 302 determines if the calibration pattern was able to be scanned correctly. If the image processor 302 determines that the calibration pattern was not able to be scanned correctly (block 508: NO), the example component interface 300 transmits a prompt to a user via the user interface 120 to display an error message (block 510). The component interface 300 transmits the prompt because if the calibration pattern was not able to be scanned correctly, then the sensor 118 is not sufficient to meet the minimum optical system specifications. As described above in conjunction with FIG. 3, the component interface 300 may take additional actions to attempt to increase the optical system specifications.

If the image processor 302 determines that the calibration pattern was able to be scanned correctly (block 508: YES), the example image processor 302 calibrates (e.g., via instructions sent by the component interface 300) the sensor 118 based on the calibration pattern (block 512). At block 514, image processor 302 (e.g., using the component interface 300) instructs the sensor 118 to scan the test grid 100. For example, the component interface 300 may prompt the user to scan (e.g., capture an image of) the test grid using the sensor 118. Thus, in this example, the test results represented by the test grid 110 are read without inserting the LFA device 104 into a reader or other machine.

At block 516, the example image processor 302 selects a target analyte corresponding to one or more test zones of the test grid 110. For example, if an LFA corresponds to diagnostic testing of three diseases, the example image processor 302 selects the target analyte for a first one of the diseases.

At block 518, the example image processor 302 identifies the target zones of the test grid pattern that correspond to the selected target

analyte. For example, using the example test grid 110 of FIG. 1B, if the image processor 302 has selected a first target analyte for a disease corresponding to test zones marked with 'x,' then the example image processor identifies the A-1, B-2, D-3, A-4, and A-5 zones as target zones that correspond to the selected target analyte (e.g., 'x'). At block 520, the example image processor 302 determines the results of the selected tests based on the identified target zones. Using the above example, the image processor 302 analyzes the optical signals (e.g., colors) of the A-1, B-2, D-3, A-4, and A-5 zones to determine the results. For example, if zones A-1, B-2, D-3, and A-4 are red and zone A-5 is white, the example image processor 302 determines a positive result for zones A-1, B-2, D-3, and A-4 and a negative result for zone A-5.

At block 522, the example comparator 308 determines if there are more than a first threshold number of zones that present positive results. The threshold number may be based on the type of analyte being analyzed, user preferences, administrator preferences, industry standards, and/or regulations. If the example comparator 208 determines that more than a threshold number of zones present positive results (block 522: YES), the comparator 208 flags the selected test for the target analyte as positive (block 524), and control continues to block 532. If the example comparator 208 determines that more than a threshold number of zones present results that are not positive (block 522: NO), the comparator 308 determines if more than a second threshold number of zone present negative results (block 526). The first threshold of block 526 may be the same or different than the second threshold of block 522. If the example comparator 308 determines that more than the threshold number of zones present negative results (block 526: YES), the example comparator 308 flags the selected test for the target analyte as negative (block 528), and control continues to block 532.

If the example comparator 308 determines that more than the threshold number of zones present results that are not negative (block 526: NO), the example comparator 308 flags the selected test for the target analyte as indeterminate (block 530). At block 532, the example image processor 302 determine if there is/are additional target analyte(s) to process. If the example image processor 302 determines that there is/are additional target analyte(s) to process (block 532: YES), control returns to block 516 of FIG. 5A to determine the result of the additional target analyte. If the example image processor 302 determines that there are no additional target analytes to process (block 532: NO), the example component interface 300 transmits instructions to the example user interface 120 to display the results based on the flag(s) (block 534).

At block 536, the example component interface 300 transmits instructions to a transmitter of the reader 114 to transmit the results and corresponding LFA identification information and/or contextual information to a monitoring entity and/or the example results storage 310 stores the results and corresponding LFA identification information and/or contextual information. If the results and corresponding information is transmitted to a monitoring entity, the monitoring entity can process the results and/or perform statistical analysis to determine whether there is an outbreak of disease, identify whether LFAs are being counterfeited, etc., based on multiple received results from one or more enhanced LFA reader applications. If a network connection is not currently present, the example component interface 300 may delay instructions to the transmitter until a network connection is established. Additionally or alternatively, the example component interface 300 may transmit results from the results storage 310 periodically, aperiodically, and/or based on a trigger (e.g., a result request from a measuring entity).

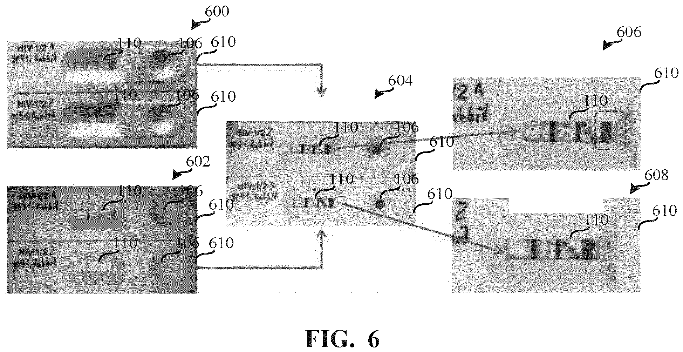

FIG. 6 illustrates an example implementation of the example enhanced LFA device 104 of FIG. 1A. FIG. 6 includes five images 600, 602, 604, 606, 608 of an example implementation of the example enhanced LFA device 104 of FIG. 1 during an example testing operation. The example images illustrate the example sample pad 106, the example test grid 110, and an example LFA housing 110. Although the illustrated example includes a particular number of test zones in a particular formation, FIG. 6 may be described in conjunction with any number of test zone in any formation.

The first image 600 of FIG. 6 (a photograph taken without a flash) includes two enhanced LFAs (e.g., that correspond to the LFA 104), and the second image 602 (a photograph taken with a flash) includes two enhanced LFAs (e.g., that corresponds to the LFA 104). The images 600, 602 are of two enhanced LFAs after a sample was applied to the sample pad 106. The line(s) on the images 600, 602 are red due to gold nanoparticles being bound to the corresponding test lines due to the target being present in the sample. The third image 604 (a photograph taken with a flash) includes the example enhanced LFAs after a sample has been applied to the example sample pad 106 and silver amplification has been used to amplify the visual cue of the result (e.g., as shown in the change of color at the sample pad 106). The third image 604 has had a silver amplification process applied to strengthen the visual indications on the example enhanced LFAs.

The fourth image 606 and fifth image 608 show a close-up view of the test grid 110 of the respective enhanced LFAs of the third image 604. The fourth image 606 illustrates the depletion effect described above in conjunction with FIG. 1A and 1B. As described above, if a depletion effect is an issue, the example test grid 110 may be structured to include a test zone for a particular analyte in one or more rows but only one column to overcome the depletion effect. Accordingly, in the example test grid 110 of FIG. 1B, each test and control zone occur in every row but only once in each column. Alternatively, the test and control zones may occur in a fewer number of rows.

FIG. 7 is a block diagram of an example processor platform 700 structured to execute the instructions of FIG. 4 to implement the test grid generator 102 of FIG. 2. The processor platform 700 can be, for example, a server, a personal computer, a workstation, a self-learning machine (e.g., a neural network), an Internet appliance, or any other type of computing device.

The processor platform 700 of the illustrated example includes a processor 712. The processor 712 of the illustrated example is hardware. For example, the processor 712 can be implemented by one or more integrated circuits, logic circuits, microprocessors, GPUs, DSPs, or controllers from any desired family or manufacturer. The hardware processor may be a semiconductor based (e.g., silicon based) device. In this example, the processor implements the example test grid applicator 200, the example user interface 202, and the example modulo determiner 204.

The processor 712 of the illustrated example includes a local memory 713 (e.g., a cache). The processor 712 of the illustrated example is in communication with a main memory including a volatile memory 714 and a non-volatile memory 716 via a bus 718. The volatile memory 714 may be implemented by Synchronous Dynamic Random Access Memory (SDRAM), Dynamic Random Access Memory (DRAM), RAMBUS^{®} Dynamic Random Access Memory (RDRAM^{®}) and/or any other type of random access memory device. The non-volatile memory 716 may be implemented by flash memory and/or any other desired type of memory device. Access to the main memory 714, 716 is controlled by a memory controller. The example local memory 713 implements the example test grid pattern storage 206.

The processor platform 700 of the illustrated example also includes an interface circuit 720. The interface circuit 720 may be implemented by any type of interface standard, such as an Ethernet interface, a universal serial bus (USB), a Bluetooth^{®} interface, a near field communication (NFC) interface, and/or a PCI express interface.

**In** the illustrated example, one or more input devices 722 are connected to the interface circuit 720. The input device(s) 722 permit(s) a user to enter data and/or commands into the processor 712. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 724 are also connected to the interface circuit 720 of the illustrated example. The output devices 724 can be implemented, for example, by display devices (e.g., a light emitting diode (LED), an organic light emitting diode (OLED), a liquid crystal display (LCD), a cathode ray tube display (CRT), an in-place switching (IPS) display, a touchscreen, etc.), a tactile output device, a printer and/or speaker. The interface circuit 720 of the illustrated example, thus, typically includes a graphics driver card, a graphics driver chip and/or a graphics driver processor.

The interface circuit 720 of the illustrated example also includes a communication device such as a transmitter, a receiver, a transceiver, a modem, a residential gateway, a wireless access point, and/or a network interface to facilitate exchange of data with external machines (e.g., computing devices of any kind) via a network 726. The communication can be via, for example, an Ethernet connection, a digital subscriber line (DSL) connection, a telephone line connection, a coaxial cable system, a satellite system, a line-of-site wireless system, a cellular telephone system, etc.

The processor platform 700 of the illustrated example also includes one or more mass storage devices 728 for storing software and/or data. Examples of such mass storage devices 728 include floppy disk drives, hard drive disks, compact disk drives, Blu-ray disk drives, redundant array of independent disks (RAID) systems, and digital versatile disk (DVD) drives.

The machine executable instructions 732 of FIG. 4 may be stored in the mass storage device 728, in the volatile memory 714, in the non-volatile memory 716, and/or on a removable non-transitory computer readable storage medium such as a CD or DVD.

FIG. 8 is a block diagram of an example processor platform 800 structured to execute the instructions of FIGS. 5A-5B to implement the enhanced LFA reader application 116 of FIG. 3. The processor platform 800 can be, for example, a personal computer, a workstation, a self-learning machine (e.g., a neural network), a mobile device (e.g., a cell phone, a smart phone, a tablet such as an iPad^{™}), a personal digital assistant (PDA), a personal video recorder, or any other type of computing device.

The processor platform 800 of the illustrated example includes a processor 812. The processor 812 of the illustrated example is hardware. For example, the processor 812 can be implemented by one or more integrated circuits, logic circuits, microprocessors, GPUs, DSPs, or controllers from any desired family or manufacturer. The hardware processor may be a semiconductor based (e.g., silicon based) device. In this example, the processor implements the example component interface 300, the example image processor 302, the example test grid determiner 304, the example modulo determiner 305, and the example comparator 308.

The processor 812 of the illustrated example includes a local memory 813 (e.g., a cache). The processor 812 of the illustrated example is in communication with a main memory including a volatile memory 814 and a non-volatile memory 816 via a bus 818. The volatile memory 814 may be implemented by SDRAM, DRAM, RDRAM^{®} and/or any other type of random access memory device. The non-volatile memory 816 may be implemented by flash memory and/or any other desired type of memory device. Access to the main memory 814, 816 is controlled by a memory controller. The example local memory 813 implements the example test grid pattern storage 306 and the example results storage 310.

The processor platform 800 of the illustrated example also includes an interface circuit 820. The interface circuit 820 may be implemented by any type of interface standard, such as an Ethernet interface, a USB, a Bluetooth^{®} interface, an NFC interface, and/or a PCI express interface.

**In** the illustrated example, one or more input devices 822 are connected to the interface circuit 820. The input device(s) 822 permit(s) a user to enter data and/or commands into the processor 812. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 824 are also connected to the interface circuit 820 of the illustrated example. The output devices 824 can be implemented, for example, by display devices (e.g., an LED, an OLED, an LCD, a CRT display, an IPS display, a touchscreen, etc.), a tactile output device, a printer and/or speaker. The interface circuit 820 of the illustrated example, thus, typically includes a graphics driver card, a graphics driver chip and/or a graphics driver processor.

The interface circuit 820 of the illustrated example also includes a communication device such as a transmitter, a receiver, a transceiver, a modem, a residential gateway, a wireless access point, and/or a network interface to facilitate exchange of data with external machines (e.g., computing devices of any kind) via a network 826. The communication can be via, for example, an Ethernet connection, a DSL connection, a telephone line connection, a coaxial cable system, a satellite system, a line-of-site wireless system, a cellular telephone system, etc.

The processor platform 800 of the illustrated example also includes one or more mass storage devices 828 for storing software and/or data. Examples of such mass storage devices 828 include floppy disk drives, hard drive disks, compact disk drives, Blu-ray disk drives, RAID systems, and DVD drives.

The machine executable instructions 832 of FIGS 5A-5B may be stored in the mass storage device 828, in the volatile memory 814, in the non-volatile memory 816, and/or on a removable non-transitory computer readable storage medium such as a CD or DVD.

From the foregoing, it will be appreciated that example methods, apparatus and articles of manufacture have been disclosed that make and/or process a diagnostic test. The disclosed methods, apparatus and articles of manufacture improve the efficiency of determining results of a diagnostic test. To reduce and/or otherwise eliminate environment light issues, examples disclosed herein add redundancy to the test zones (e.g., targets) of a diagnostic device. Additionally, examples disclosed herein can utilize the calibration image and/or pattern to verify whether the smartphone sensing the calibration image and/or pattern meets the validation requirements. Further, examples disclosed herein integrate security features in the enhanced diagnostic device to reduce counterfeiting. The disclosed methods, apparatus and articles of manufacture are accordingly directed to one or more improvement(s) in the functioning of an diagnostic devce and diagnostic device readers.

## Claims

1. A device for use with a fluid sample, the device comprising:
a conjugate pad including conjugates for a target analyte; and
a test grid including a plurality of zones in a two-dimensional grid, the test grid including:
a first test zone in the plurality of zones including at least one of first immobilized antibodies or first immobilized antigens to attach to the target analyte labeled with a first conjugate of the conjugates;
a second test zone in the plurality of zones including at least one of second immobilized antibodies or second immobilized antigens to attach to the target analyte labeled with a second conjugate of the conjugates;
a control zone in the plurality of zones including at least one of third immobilized antibodies or third immobilized antigens to attach to conjugates not bound to the target analyte; and
a blank zone in the plurality of zones that does not include immobilized antibodies or immobilized antigens;
wherein a location of the first test zone and the second test zone within the test grid is according to a test grid pattern that is related to identifying information of the device, wherein in the test grid pattern the test zones, control zone and blank zone are randomly distributed such that results appear random to a user.

2. The device of claim 1, wherein the identifying information is included on a housing of the device, optionally in the form of a code printed on a housing of the device.

3. The device of claim 1 or 2, wherein the target analyte is a first target analyte, the conjugates are first conjugates, and the conjugate pad includes second conjugates for a second target analyte,
wherein the test grid includes a third test zone in the plurality of zones including at least one of third immobilized antibodies or third immobilized antigens to attach to the second target analyte labeled with a conjugate of the second conjugates.

4. A method of generating a testing grid of a device according to any preceding claim, the method comprising:
obtaining a code corresponding to identification information on the device;
determining a test grid pattern value based on the identification information; and
applying at least one of immobilized antibodies or immobilized antigens corresponding to a target analyte to a zone in a two dimensional test grid on a porous membrane of the device according to a test grid pattern corresponding to the test grid pattern value.

5. The method of claim 4, wherein the code corresponds to at least one of a production date, an expiry date, or a production identifier.

6. The method of claim 4 or 5, further including calculating a modulo based on the identification information to determine the test grid pattern value.

7. The method of any of claims 4 to 6, further including applying a calibration pattern to a housing of the device.

8. The method of any of claims 5 to 7, wherein different identification information corresponds to different test grid patterns.

9. The method of any of claims 5 to 8, wherein the test grid pattern corresponds to a number of target analytes and a number of parts per line, a total number of different combinations for the testing grid pattern of the two dimensional grid being equal to a factorial of the number of parts per line to a power of the number of the target analytes.

10. A method for reading a fluid sample on a device according to any one of claims 1 to 4, the method comprising:
obtaining a code corresponding to a device;
obtaining test zone information related to test zones based on the code;
identifying target zones that correspond to a target analyte based on the test zone information; and
determining a result corresponding to a presence of the target analyte based on a presence or an absence of an optical signal in respective ones of the target zones in an image of a test grid of the device.

11. The method of claim 10, further including performing a modulo operation on the code to determine a value, the test zone information corresponding to the value.

12. The method of claim 10 or 11, further including:
processing an image of a calibration pattern on a housing of the device; and
assessing a calibration of the device based on the calibration pattern;
optionally further including preventing the determining of the result when the calibration indicates that the device is not calibrated.

13. The method of any of claims 10 to 12, wherein the optical signal includes a color, wherein the determining of the result includes:
when more than a first threshold of the target zones in the image correspond to a first color, determining a positive result; and
when more than a second threshold of the target zones in the image correspond to a second color, determining a negative result.

14. The method of any of claims 10 to 13, further including:
obtaining the image using a sensor of a mobile device, the mobile device determining the result; and
displaying the result on a display of the mobile device.

15. The method of any of claims 10 to 14, wherein one or more of:
(A) the obtaining of the test information includes selecting a test grid pattern corresponding to the code; and
(B) the code is obtained from a user interface or a sensor.

## Patentansprüche

1. Vorrichtung zur Verwendung mit einer Fluidprobe, wobei die Vorrichtung umfasst:
ein Konjugatpad mit Konjugaten für einen Zielanalyten; und
ein Testraster mit einer Vielzahl von Zonen in einem zweidimensionalen Raster, wobei das Testraster Folgendes umfasst:
eine erste Testzone in der Vielzahl von Zonen, die mindestens einen der ersten immobilisierten Antikörper oder eines der ersten immobilisierten Antigene enthält, um an den Zielanalyten zu binden, der mit einem ersten Konjugat der Konjugate markiert ist;
eine zweite Testzone in der Vielzahl von Zonen, die mindestens einen der zweiten immobilisierten Antikörper oder eines der zweiten immobilisierten Antigene enthält, um an den Zielanalyten zu binden, der mit einem zweiten Konjugat der Konjugate markiert ist;
eine Kontrollzone in der Vielzahl von Zonen, die mindestens einen dritten immobilisierten Antikörper oder ein drittes immobilisiertes Antigen enthält, um an Konjugate zu binden, die nicht an den Zielanalyten gebunden sind; und
eine Leerzone in der Vielzahl von Zonen, die keine immobilisierten Antikörper oder immobilisierten Antigene enthält;
wobei eine Position der ersten Testzone und der zweiten Testzone innerhalb des Testrasters einem Testrastermuster entspricht, das sich auf identifizierende Informationen der Vorrichtung bezieht, wobei in dem Testrastermuster die Testzonen, die Kontrollzone und die Leerzone zufällig verteilt sind, sodass die Ergebnisse für einen Benutzer zufällig erscheinen.

2. Vorrichtung nach Anspruch 1, wobei die identifizierenden Informationen auf einem Gehäuse der Vorrichtung enthalten sind, optional in Form eines Codes, der auf ein Gehäuse der Vorrichtung gedruckt ist.

3. Vorrichtung nach Anspruch 1 oder 2, wobei der Zielanalyt ein erster Zielanalyt ist, die Konjugate erste Konjugate sind und das Konjugatpad zweite Konjugate für einen zweiten Zielanalyten enthält,
wobei das Testraster eine dritte Testzone in der Vielzahl von Zonen enthält, die mindestens einen der dritten immobilisierten Antikörper oder eines der dritten immobilisierten Antigene enthält, um an den zweiten Zielanalyten zu binden, der mit einem Konjugat der zweiten Konjugate markiert ist.

4. Verfahren zum Erzeugen eines Testrasters einer Vorrichtung gemäß einem der vorhergehenden Ansprüche, wobei das Verfahren Folgendes umfasst:
Abrufen eines Codes, der Identifikationsinformationen über die Vorrichtung entspricht;
Bestimmen eines Werts des Testrastermusters anhand der Identifikationsinformationen; und
Aufbringen von mindestens einem der immobilisierten Antikörper oder immobilisierten Antigene, die einem Zielanalyten entsprechen, auf eine Zone in einem zweidimensionalen Testraster auf einer porösen Membran der Vorrichtung entsprechend einem Testrastermuster, das dem Wert des Testrastermusters entspricht.

5. Verfahren nach Anspruch 4, wobei der Code mindestens einem Produktionsdatum, einem Verfallsdatum oder einer Produktionskennung entspricht.

6. Verfahren nach Anspruch 4 oder 5, das ferner das Berechnen eines Modulus auf Basis der Identifikationsinformationen beinhaltet, um den Wert des Testrastermusters zu bestimmen.

7. Verfahren nach einem der Ansprüche 4 bis 6, das ferner das Aufbringen eines Kalibrationsmusters auf ein Gehäuse der Vorrichtung umfasst.

8. Verfahren nach einem der Ansprüche 5 bis 7, wobei unterschiedliche Identifikationsinformationen unterschiedlichen Testrastermustern entsprechen.

9. Verfahren nach einem der Ansprüche 5 bis 8, wobei das Testrastermuster einer Anzahl von Zielanalyten und einer Anzahl von Teilen pro Linie entspricht, wobei eine Gesamtzahl verschiedener Kombinationen für das Testrastermuster des zweidimensionalen Rasters gleich einem Faktor der Anzahl von Teilen pro Linie hoch der Anzahl der Zielanalyten ist.

10. Verfahren zum Auslesen einer Fluidprobe auf einer Vorrichtung gemäß einem der Ansprüche 1 bis 4, wobei das Verfahren umfasst:
Abrufen eines Codes, der einer Vorrichtung entspricht;
Abrufen von Testzoneninformationen zu Testzonen basierend auf dem Code;
Identifizieren von Zielzonen, die einem Zielanalyten entsprechen, anhand der Testzoneninformationen; und
Bestimmen eines Ergebnisses, das einem Vorhandensein des Zielanalyten entspricht, auf Basis eines Vorhandenseins oder eines Fehlens eines optischen Signals in jeweiligen der Zielzonen in einem Bild eines Testrasters der Vorrichtung.

11. Verfahren nach Anspruch 10, das ferner das Durchführen einer Modulo-Operation an dem Code umfasst, um einen Wert zu bestimmen, wobei die Testzoneninformationen dem Wert entsprechen.

12. Verfahren nach Anspruch 10 oder 11, das ferner umfasst:
Verarbeiten eines Bildes eines Kalibrationsmusters auf einem Gehäuse der Vorrichtung; und
Beurteilen einer Kalibrierung der Vorrichtung basierend auf dem Kalibrationsmuster;
das optional ferner das Verhindern der Bestimmung des Ergebnisses umfasst, wenn die Kalibrierung anzeigt, dass die Vorrichtung nicht kalibriert ist.

13. Verfahren nach einem der Ansprüche 10 bis 12, wobei das optische Signal eine Farbe enthält, wobei das Bestimmen des Ergebnisses umfasst:
wenn mehr als ein erster Schwellenwert der Zielzonen in dem Bild einer ersten Farbe entsprechen, das Bestimmen eines positiven Ergebnisses; und
wenn mehr als ein zweiter Schwellenwert der Zielzonen in dem Bild einer zweiten Farbe entsprechen, das Bestimmen eines negativen Ergebnisses.

14. Verfahren nach einem der Ansprüche 10 bis 13, das ferner umfasst:
Erfassen des Bildes unter Verwendung eines Sensors einer mobilen Vorrichtung, wobei die mobile Vorrichtung das Ergebnis bestimmt; und
Anzeigen des Ergebnisses auf einem Display der mobilen Vorrichtung.

15. Verfahren nach einem der Ansprüche 10 bis 14, wobei eines oder mehrere von:
(A) dem Abrufen der Testinformationen das Auswählen eines Testrastermusters entsprechend dem Code beinhaltet; und
(B) wobei der Code von einer Benutzeroberfläche oder einem Sensor abgerufen wird.

## Revendications

1. Dispositif destiné à une utilisation avec un échantillon de fluide, le dispositif comprenant :
un tampon de conjugués comprenant des conjugués pour un analyte cible ; et
une grille de test comprenant une pluralité de zones dans une grille bidimensionnelle, la grille de test comprenant :
une première zone de test dans la pluralité de zones comprenant au moins l'un de premiers anticorps immobilisés ou de premiers antigènes immobilisés à fixer à l'analyte cible marqué avec un premier conjugué des conjugués ;
une deuxième zone de test dans la pluralité de zones comprenant au moins l'un de deuxièmes anticorps immobilisés ou de deuxièmes antigènes immobilisés à fixer à l'analyte cible marqué avec un deuxième conjugué des conjugués ;
une zone de contrôle dans la pluralité de zones comprenant au moins l'un de troisièmes anticorps immobilisés ou de troisièmes antigènes immobilisés à fixer à des conjugués non liés à l'analyte cible ; et
une zone vide dans la pluralité de zones qui ne comprend pas d'anticorps immobilisés ou d'antigènes immobilisés ;
dans lequel un emplacement de la première zone de test et de la deuxième zone de test à l'intérieur de la grille de test est conforme à un motif de grille de test qui correspond à des informations d'identification du dispositif, dans lequel, dans le motif de grille de test, les zones de test, la zone de contrôle et la zone vide sont réparties de manière aléatoire de telle sorte que les résultats apparaissent aléatoires à un utilisateur.

2. Dispositif selon la revendication 1, dans lequel les informations d'identification sont incluses sur un boîtier du dispositif, optionnellement sous la forme d'un code imprimé sur un boîtier du dispositif.

3. Dispositif selon la revendication 1 ou 2, dans lequel l'analyte cible est un premier analyte cible, les conjugués sont des premiers conjugués, et le tampon de conjugués comprend des deuxièmes conjugués pour un deuxième analyte cible,
dans lequel la grille de test comprend une troisième zone de test dans la pluralité de zones comprenant au moins l'un de troisièmes anticorps immobilisés ou de troisièmes antigènes immobilisés à fixer au deuxième analyte cible marqué avec un conjugué des deuxièmes conjugués.

4. Procédé de production d'une grille de test d'un dispositif selon l'une quelconque des revendications précédentes, le procédé comprenant :
l'obtention d'un code correspondant à des informations d'identification sur le dispositif ;
la détermination d'une valeur de motif de grille de test sur la base des informations d'identification ; et
l'application d'au moins l'un d'anticorps immobilisés ou d'antigènes immobilisés correspondant à un analyte cible à une zone dans une grille de test bidimensionnelle sur une membrane poreuse du dispositif selon un motif de grille de test correspondant à la valeur de motif de grille de test.

5. Procédé selon la revendication 4, dans lequel le code correspond à au moins l'un d'une date de fabrication, d'une date de péremption, ou d'un identificateur de fabrication.

6. Procédé selon la revendication 4 ou 5, comprenant en outre le calcul d'un modulo sur la base des informations d'identification pour déterminer la valeur de motif de grille de test.

7. Procédé selon l'une quelconque des revendications 4 à 6, comprenant en outre l'application d'un motif d'étalonnage sur un boîtier du dispositif.

8. Procédé selon l'une quelconque des revendications 5 à 7, dans lequel différentes informations d'identification correspondent à différents motifs de grille de test.

9. Procédé selon l'une quelconque des revendications 5 à 8, dans lequel le motif de grille de test correspond à un nombre d'analytes cibles et à un nombre de parties par ligne, un nombre total de différentes combinaisons pour le motif de grille de test de la grille bidimensionnelle étant égal à une factorielle du nombre de parties par ligne à une puissance du nombre des analytes cibles.

10. Procédé de lecture d'un échantillon de fluide sur un dispositif selon l'une quelconque des revendications 1 à 4, le procédé comprenant :
l'obtention d'un code correspondant à un dispositif ;
l'obtention d'informations sur les zones de test correspondant aux zones de tests sur la base du code ;
l'identification de zones cibles qui correspondent à un analyte cible sur la base des informations sur les zones de test ; et
la détermination d'un résultat correspondant à une présence de l'analyte cible sur la base d'une présence ou d'une absence d'un signal optique dans des zones cibles respectives des zones cibles dans une image d'une grille de test du dispositif.

11. Procédé selon la revendication 10, comprenant en outre l'exécution d'une opération modulo sur le code pour déterminer une valeur, les informations sur les zones de test correspondant à la valeur.

12. Procédé selon la revendication 10 ou 11, comprenant en outre :
le traitement d'une image d'un motif d'étalonnage sur un boîtier du dispositif ; et
l'évaluation d'un étalonnage du dispositif sur la base du motif d'étalonnage ;
comprenant optionnellement en outre le fait d'empêcher la détermination du résultat lorsque l'étalonnage indique que le dispositif n'est pas étalonné.

13. Procédé selon l'une quelconque des revendications 10 à 12, dans lequel le signal optique comprend une couleur, dans lequel la détermination du résultat comprend :
lorsque plus d'un premier seuil des zones cibles dans l'image correspondent à une première couleur, la détermination qu'un résultat est positif ; et
lorsque plus d'un deuxième seuil des zones cibles dans l'image correspondent à une deuxième couleur, la détermination qu'un résultat est négatif.

14. Procédé selon l'une quelconque des revendications 10 à 13, comprenant en outre :
l'obtention de l'image à l'aide d'un capteur d'un dispositif mobile, le dispositif mobile déterminant le résultat ; et
l'affichage du résultat sur un affichage du dispositif mobile.

15. Procédé selon l'une quelconque des revendications 10 à 14, comprenant une ou plusieurs des caractéristiques suivantes :
(A) l'obtention des informations de test comprend la sélection d'un motif de grille de test correspondant au code ; et
(B) le code est obtenu à partir d'une interface utilisateur ou d'un capteur.
